(19) Europäisches Patentamt
European Patent Office
Office européen des brevets

(11) Veröffentlichungsnummer: **0 321 871 B1**

(12) **EUROPÄISCHE PATENTSCHRIFT**

(45) Veröffentlichungstag der Patentschrift: **07.07.93**

(51) Int. Cl.5: **C07H 15/203**, C07H 17/00, C12Q 1/40

(21) Anmeldenummer: **88121068.6**

(22) Anmeldetag: **16.12.88**

# (54) Substrate für die alpha-Amylasebestimmung.

(30) Priorität: **23.12.87 DE 3743908**

(43) Veröffentlichungstag der Anmeldung:
**28.06.89 Patentblatt 89/26**

(45) Bekanntmachung des Hinweises auf die Patenterteilung:
**07.07.93 Patentblatt 93/27**

(84) Benannte Vertragsstaaten:
**AT BE CH DE ES FR GB GR IT LI LU NL SE**

(56) Entgegenhaltungen:
**EP-A- 0 085 348**
**EP-A- 0 135 758**
**EP-A- 0 156 347**

(73) Patentinhaber: **BOEHRINGER MANNHEIM GMBH**
**Sandhofer Strasse 116**
**W-6800 Mannheim 31(DE)**

(72) Erfinder: **Schmidt, Axel, Dr.**
**Gudrunstrasse 14**
**W-8000 München 19(DE)**
Erfinder: **von der Eltz, Herbert, Dr. rer. nat.**
**In der Au 21**
**W-8120 Weilheim(DE)**
Erfinder: **Rauscher, Elli, Dr. rer. nat.**
**Guardinistrasse 71**
**W-8000 München 70(DE)**

**EP 0 321 871 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue Oligoglucosidderivate, ihre Herstellung sowie ihre Verwendung als Substrate für die Bestimmung der α-Amylase.

Die Bestimmung von α-Amylase in Serum und Urin ist ein wichtiger klinischer Parameter zur Überprüfung der Bauchspeicheldrüsenfunktion. Bei den gängigsten Verfahren zur α-Amylasebestimmung werden als Substrate Oligoglucoside verwendet, welche aus drei bis acht 1,4-α-verknüpften Glucoseeinheiten bestehen, in 1-Position am reduzierenden Ende mit einer bestimmbaren Gruppe und am anderen Ende in 6- und ggf. 4-Position mit einer Schutzgruppe derivatisiert sind.

EP 135 758 beschreibt derartige Substrate, derivatisiert mit einem bestimmbaren Rest wie beispielsweise der Nitrophenylgruppe in 1-Position am reduzierenden Ende und einer Schutzgruppe in der 4- und 6-Position am anderen Ende. Als Schutzgruppe geeignet sind beispielsweise geradkettige oder verzweigte Alkyl- oder Alkoylgruppen, ein Phenylrest oder eine Ethylidenbrücke.

Die Durchführung einer α-Amylasebestimmung mit derartigen Substraten als enzymatischer Farbtest ist für das Substrat 4,6-Ethyliden-p-nitrophenyl-α-D-maltoheptaosid (Et-$G_7$-PNP) in Fresenius Z. Anal. Chem. 324 (1986) 3O4-3O5 beschrieben. Dabei erfolgt die Bestimmung nach folgendem Testprinzip (vereinfacht):

$$5\ Et\text{-}G_7\text{-}PNP + 5\ H_2O \xrightarrow{\alpha\text{-Amylase}}$$

$$2\ G_2\text{-}PNP + 2\ G_3\text{-}PNP + G_4\text{-}PNP + 2\ Et\text{-}G_5 + 2\ Et\text{-}G_4 + Et\text{-}G_3$$

$$2\ G_2\text{-}PNP + 2\ G_3\text{-}PNP + 10\ H_2O \xrightarrow{\alpha\text{-Glucosidase}}$$

$$4\ PNP + 10\ G$$

(Et = Ethyliden, G = Glucose,
PNP = p-Nitrophenol)

Der Vorteil dieser Substrate liegt insbesondere darin, daß die Hilfsenzyme, die zur Freisetzung des bestimmbaren Rests verwendet werden, wie z. B. α-Glucosidase oder β-Glucosidase, nur das bereits durch α-Amylase gespaltene Substrat, nicht jedoch das nichtgespaltene Substrat angreifen.

Damit wird mit den geschützten Substraten im Vergleich zu den ungeschützten Substraten eine verbesserte Lagerfähigkeit der Reagenzienmischung erreicht.

Zur Erhöhung der Präzision ist es jedoch, insbesondere bei der Bestimmung von geringen α-Amylase-Aktivitäten, wünschenswert, α-Amylase-Substrate mit einer noch höheren Empfindlichkeit im photometrischen Test als dies mit den bekannten Substraten erreicht werden kann, zu erhalten. Unter Empfindlichkeit wird hier das Verhältnis α-Amylase-Aktivität zur Extinktionszunahme pro Zeiteinheit (ΔE/min) verstanden. Eine Steigerung der Empfindlichkeit bedeutet also, daß bei gleicher α-Amylaseaktivität der Probe ein größeres ΔE/min gefunden wird.

Aufgabe der vorliegenden Erfindung ist es daher, α-Amylase-Substrate bereitzustellen, die lagerfähig sind und eine verbesserte Empfindlichkeit zeigen.

Gelöst wird diese Aufgabe erfindungsgemäß durch Verbindungen der allgemeinen Formel I

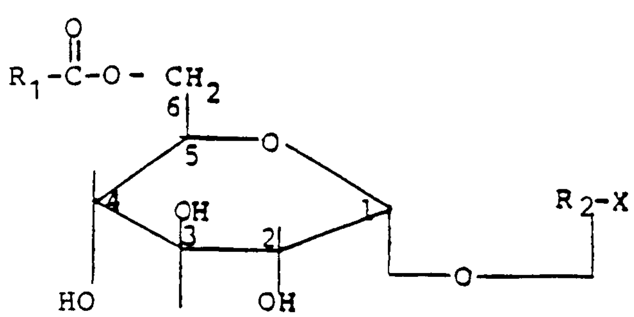

(I)

in der $R_1$ ein Wasserstoffatom, eine Methyl-, Ethyl-, Propyl-, Isopropyl-, Butyl-, tert. Butyl-, 1-Alkoxyalkyl oder eine gegebenenfalls hydrophil substituierte Cyclopropyl-, Cyclobutyl-, Cyclopentyl-, Cyclohexyl-, Phenyl-, Tetrahydropyranyl-, Piperidinyl-, gegebenenfalls N-Methyl- oder Ethyl-substituiert, Pyridinyl-, Thiophenyl- oder 1.1.-Dioxo-tetrahydrothiopyranylgruppe oder eine Aminogruppe, die gegebenenfalls gleich oder verschieden substituiert ist mit einer Methyl-, Ethyl-, Propyl- oder Isopropylgruppe, $R_2$ einen Oligoglucosidrest mit 2,3 oder 4 Glucoseeinheiten und X ein Wasserstoffatom oder einen optisch bestimmbaren Rest darstellen.

Überraschenderweise hat sich gezeigt, daß die erfindungsgemäßen Substrate mit den oben genannten Schutzgruppen für die terminale C6-Hydroxygruppe deutlich empfindlicher als die bekannten Substrate sind und damit die Präzision der α-Amylase-Bestimmung wesentlich verbessert werden kann.

Als $R_1$ ist eine Methyl-, Isopropyl- oder eine gegebenenfalls hydrophil substituierte Cyclopropylgruppe bevorzugt. Die Isopropyl-und Cyclopropylgruppe ist besonders bevorzugt. Als $R_2$ ist ein Oligoglucosidrest mit 4 Glucoseeinheiten bevorzugt.

Als hydrophile Substituenten sind beispielsweise geeignet Carboxy-, Hydroxy-, Sulfonsäure-, Dimethylamino-, Phosphat-, Halogen- und/oder Nitrogruppen.

Der optisch bestimmbare Rest in 1-Position am reduzierenden Ende des Substrats kann sowohl in α-Verknüpfung oder in β-Verknüpfung gebunden sein.

Falls X ein optisch bestimmbarer Rest ist, kann es sich um einen Rest handeln, der selbst im sichtbaren- oder UV-Bereich eine Färbung aufweist, oder um einen Rest der erst nach Umsetzung mit einer weiteren Verbindung optisch bestimmbar wird, beispielsweise durch Überführen in einen Farbstoff oder durch Kopplung mit einem Farbstoff. Derartige optisch bestimmbare Reste sind dem Fachmann geläufig und bedürfen hier keiner näheren Erläuterung. Bevorzugt sind Nitrogruppenhaltige und ggf. chlorierte Phenylreste, wie die Nitrophenyl-, die 3,4-Di-nitrophenyl- oder die 2-Chloro-4-nitrophenylgruppe sowie die Resorufingruppe und deren Derivate.

Die Herstellung der erfindungsgemäßen Verbindungen und der Vergleichsverbindungen erfolgt beispielsweise analog zu den in EP 135 758 beschriebenen Verfahren. Es kann auch in das ungeschützte Substrat die gewünschte Schutzgruppe über aktivierte Carbonsäuregruppen wie z. B. über die entsprechenden Orthoester, Säurechloride, Anhydride, aus aktivierten Estern enzymatisch (JACS 110 (1988) 584 - 589), aus Acetalen oder direkt aus der Carbonsäure über wasserentziehende Mittel wie z. B. durch die Mitsunobu-Reaktion (Poster von S. Czernecki auf dem XIVth International Carbohydrate Symposium (1988), Stockholm; Synthesis 1981, S. 1 - 28) eingeführt werden. Besonders bevorzugt ist die Herstellung über die entsprechenden Orthoester als Zwischenprodukte und die Herstellung über die Mitsunobu-Reaktion. Die Orthoester werden bevorzugt aus den entsprechenden Nitrilen hergestellt (vgl. z. B. Houben-Weyl, Band VI/3 (1965) 300 - 313). Die Nitrile können beispielsweise aus den entsprechenden Carbonsäuren hergestellt werden. Die Reinigung der geschützte Substrate kann zum Beispiel chromatographisch wie über Ionentauscher oder MPLC erfolgen.

Ein weiterer Gegenstand der Erfindung ist ein Verfahren zur Herstellung der Verbindungen der allgemeinen Formel I, welches dadurch gekennzeichnet ist, daß man Verbindungen der allgemeinen Formel II

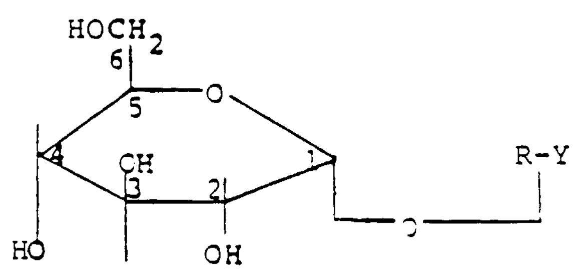

(II)

in der R ein Oligoglucosidrest mit 2,3 oder 4 Glucoseeinheiten und Y ein Wasserstoffatom oder einen optisch bestimmbaren Rest darstellen, mit Carbonsäuren, deren Estern, die ggf. zusätzlich aktiviert sein können, oder Orthoestern der allgemeinen Formel III

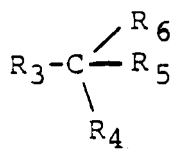

(III)

in der $R_3$ ein Wasserstoffatom, eine Methyl-, Ethyl-, Propyl-, Isopropyl-, Butyl-, tert. Butyl-, 1-Alkoxyalkyl- oder gegebenenfalls eine hydrophil substituierte Cyclopropyl-, Cyclobutyl-, Cyclopentyl., Cyclohexyl-, Phenyl-, Tetrahydropyranyl-, Piperidinyl-, ggf. N-Methyl- oder Ethyl-substituiert, Pyridinyl-, Thiophenyl-, 1.1.-Dioxo-tetrahydrothiopyranyl-Gruppe oder eine Aminogruppe, die ggf. gleich oder verschieden substituiert ist mit einer Methyl-, Ethyl-, Propyl- oder Isopropylgruppe, $R_4$ eine Hydroxy-, eine Alkoxy-, eine Dimethyl- oder Diethyl-substituierte Aminogruppe und $R_5$ and $R_6$, die gleich oder verschieden sein können, eine Alkoxygruppe mit 1 bis 4 Kohlenstoffatomen oder zusammen ein Sauerstoffatom darstellen, in absolutem Lösungsmittel unter Feuchtigkeitsausschluß und Säurekatalyse oder in Gegenwart eines wasserentziehenden Mittels umsetzt, ggf. hydrolysiert und das Produktgemisch anschließend beispielsweise chromatographisch auftrennt. Bevorzugt wird die Reaktion bei 20 bis 50 °C durchgeführt.

Auf diese Weise können beispielsweise Verbindungen der allgemeinen Formel I, in der $R_1$ eine Methyl-, eine Isopropyl- oder eine hydrophil substituierte Cyclopropylgruppe, $R_2$ ein Oligoglucosid mit 4 Glucoseeinheiten und X ein Resorufin- oder p-Nitrophenylrest bedeuten, erhalten werden, wenn man Verbindungen der allgemeinen Formel II, in der R und Y die zu $R_2$ und X analoge Bedeutung haben, mit der mindestens 4fach äquimolaren Menge eines Orthoesters der allgemeinen Formel III, in der $R_3$ eine Methyl-, Isopropyl- oder eine hydrophil substituierte Cyclopropylgruppe, $R_4$ eine Methoxy- oder Ethoxygruppe und $R_5$ und $R_6$ entweder beide eine Methoxy-, oder beide eine Ethoxygruppe bedeuten, in absolutem Lösungsmittel löst und 1 Mol p-Toluolsulfonsäure unter Feuchtigkeitsausschluß einrührt.

Analog können auch mit der entsprechenden Menge eines Acetals und Verbindungen der allgemeinen Formel III beispielsweise 4,6-Ethyliden-Resorufinyl-$\beta$-D- bzw. 4,6-Ethyliden-p-Nitrophenyl-$\alpha$-D-maltepentaoside erhalten werden. Anstatt p-Toluolsulfonsäure können auch andere organische Säuren sowie Mineralsäuren und/oder Lewis-Säuren zur Säurekatalyse eingesetzt werden.

Ebenso bevorzugt ist die Herstellung der erfindungsgemäßen Verbindungen aus Carbonsäuren gemäß der allgemeinen Formel III, in der beispielsweise $R_3$ eine Methyl-, eine Isopropyl- oder eine hydrophil substituierte Cyclopropylgruppe, $R_4$ eine Hydroxy- oder eine Alkoxygruppe und $R_5$ und $R_6$ zusammen ein Sauerstoffatom darstellen, und Verbindungen der allgemeinen Formel II, die unter Feuchtigkeitsausschluß und in Gegenwart eines oder mehrerer wasserentziehender Mittel zur Reaktion gebracht werden. Als wasserentziehendes Mittel können beispielsweise Triphenylphosphin und/oder Diethylazodicarboxylat und/oder analog verwendbare Verbindungen verwendet werden.

Unter "Alkoxyalkyl" in der Definition von $R_1$ und $R_3$ ist eine an eine Alkylgruppe gebundene Alkoxygruppe zu verstehen, wobei jede 1 bis 6, vorzugsweise 1 bis 4 Kohlenstoffatome aufweist.

Die "Alkoxygruppe" in der Definition von $R_4$ enthält 1 bis 10, bevorzugt 1 bis 6 Kohlenstoffatome und ggf. ein oder mehrere Heteroatome, bevorzugt Stickstoff oder Chlor und kann geradkettig, verzweigt oder als Ring vorliegen.

Die Herstellung kann auch durch Peracetylierung des jeweiligen Glucosides (Maltotriosid, Maltotetraosid, Maltopentaosid) mit Acetanhydrid oder Acetylchlorid (Chem. Ber. 13 (188O) 267) und durch Hydrolyse der 1-ständigen Acetoxygruppe (Chem. Ber. 86 (1953) 6O4) zum Hydroxid oder Bromid erfolgen. Anschließend wird der freie Farbstoff an das Glucosid über die Chloracetimidat-Methode (Synthesis (1981) 885 - 887) oder die Königs-Knorr-Methode (J. Am. Chem. Soc. 51 (1929) 183O, Angew. Chemie 94 (1982) 184) gekuppelt. Die Kupplung kann auch direkt durch Umsetzung der jeweiligen peracetylierten Verbindung mit einen phenolischen Chromogen unter Lewis-Säure-Katalyse erfolgen (JP-OS 62/289595). Nach Kupplung des Farbstoffs wird deacetyliert und die Schutzgruppe an die endständige 6-Position über die entsprechenden Orthoester, enzymatisch oder über aktivierte Carbonsäurederivate wie Aktivester, Säurechloride oder Anhydride gekuppelt (Tetrahedron Letters 28 (1987) 38O9 - 3812, J. Am. Chem. Soc. 108 (1986) 5638). Die Vergleichsverbindungen können analog hergestellt werden.

Ein weiterer Gegenstand der Erfindung ist ein Verfahren zur Bestimmung der $\alpha$-Amylase in einer Probe durch Umsetzung mit einem Oligosaccharidsubstrat, $\alpha$-Glucosidase und/oder $\beta$-Glucosidase und Bestimmung der Spaltprodukte, dadurch gekennzeichnet, daß als Oligoglucosidsubstrat eine Verbindung der allgemeinen Formel I

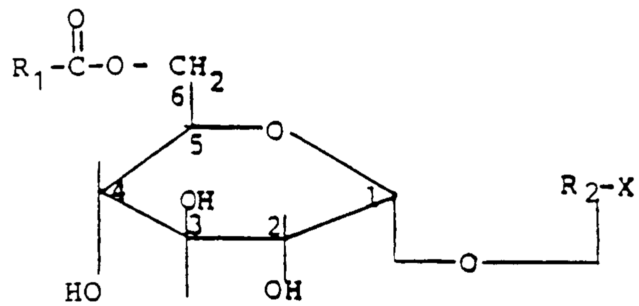

(I)

verwendet wird.

Sofern X = H ist, kann die Bestimmung der Spaltprodukte auf die dem Fachmann geläufige Weise, wie z. B. in De 27 41 192 beschrieben, erfolgen. Die Bestimmung der Spaltprodukte für den Fall, daß X ein optisch bestimmbarer Rest ist, ist dem Fachmann ebenfalls geläufig und beispielsweise in EP 135 758 beschrieben. In einer weiteren Ausführungsform wird Glucoamylase zusätzlich zugesetzt.

Ein weiterer Gegenstand der Erfindung ist ein Reagenz zur Bestimmung von α-Amylase, bestehend aus (Endkonzentrationen im Test):

O,5 - 2 mmol/l erfindungsgemäßes Substrat
3O - 1OO mmol/l NaCl
2O - 5O U/l α-Glucosidase und/oder
O,5 - 2 U/ml β-Glucosidase

Die Bestimmung wird üblicherweise in Puffer, vorzugsweise GOOD-Puffer, pH 6 - 8, vorzugsweise pH 6,5 - 7,5 und einer Konzentration von 20 - 200 mmol/l, bevorzugt von 50 - 150 mmol/l durchgeführt. Gegebenenfalls können 5 - 2O U/ml Glucoamylase zugesetzt werden. Vorzugsweise wird die Bestimmung in Gegenwart von 5-2O mmol/l $MgCl_2$ durchgeführt.

Die folgenden Beispiele und Zeichnungen erläutern die Erfindung weiter:

## Beispiel 1

### Peracetyl-β-D-maltopentaosid

100 g (0.12 mol) Maltopentaose und 81,8 g (1.0 mol) wasserfreies Natriumacetat werden in 1.1 l (11.7 mol) Acetanhydrid suspendiert und langsam unter Feuchtigkeitsausschluß bis zum Reaktionsbeginn (ca. 110 °C) aufgeheizt. Dann wird mit Eiswasser gekühlt, bis die Reaktion abgeklungen ist und anschließend zur vollständigen Umsetzung noch 1 h am Rückfluß gekocht. Das Reaktionsgemisch wird auf ca. 70 °C abgekühlt und auf 4 l Eiswasser gegossen. Nach 60 min Rühren wird der Überstand abgegossen und der Rückstand in 500 ml $CH_2Cl_2$ gelöst. Die $CH_2Cl_2$-Phase wird sukzessiv mit $H_2O$, gesättigter $NaHCO_3$-Lösung und $H_20$ ausgeschüttelt und über $Na_2SO_4$ getrocknet. Nach Abdestillieren des Lösungsmittels im Vakuum wird der Rückstand aus 1.5 l Ethanol-Isopropanol (1 : 1) unter Aktivkohlezusatz umkristallisiert.

| | |
|---|---|
| Ausbeute: | 166.2 g (89.8 % d. Th.) farblose Kristalle |
| Fp: | 120 - 125 °C; $\alpha_D^{20}$ = + 123.5 ° |
| DC: | Kieselgel 60-Platten $F_{254}$ (Merck) |
| | Toluol/Aceton (7/4) |
| | rf = 0.52 |
| $^1$H-NMR (DMSO-$d_6$): | 1.8 - 2.2 (s, 51H, $CH_3CO$); |
| | 3.8 - 5.5 (m, 35H, H-1 - H-6) |

Literatur:

Herzfeld, Chem. Ber. 13 (1880), 267

Folgende Peracetyl-β-D-Maltooligosaccharide wurden wie oben beschrieben synthetisiert, wobei anstelle von Maltopentaose gleiche Mengen von Maltose, Maltotriose, Maltotetraose, Maltohexaose und Maltoheptaose eingesetzt wurden.

| Zucker | Fp | Ausbeute ($CHCl_3$,$\alpha_D^{20}$ ) | rf (Kieselgel 60; Toluol : Aceton = 7 : 4) |
|---|---|---|---|
| Maltose | 159-160°C | 90 % + 62.5° | 0,64 |
| Maltotriose | - | 85 % + 100.3° | 0,59 |
| Maltotetraose | - | 88 % + 109.7° | 0,56 |
| Maltopentaose | 120-125°C | 90 % + 123,5° | 0,52 |
| Maltohexaose | 140-145°C | 91 % + 130° | 0,49 |
| Maltoheptaose | 160°C | 82 % - | 0,46 |

**Beispeil 2**

**Hydroxy-α-D-Peracetylmaltopentaosid**

150 g (0.096 mol) Peracetyl-β-D-maltopentaosid werden in 200 ml abs. Tetrahydrofuran gelöst. Dazu gibt man unter Rühren 33 ml (0.3 mol) Benzylamin und läßt 2 h bei Raumtemperatur unter Feuchtigkeitsausschluß rühren. Anschließend wird bis zur Trockene im Vakuum eingeengt, der Rückstand in 400 ml $CH_2Cl_2$ gelöst und die $CH_2Cl_2$-Phase nacheinander mit je 400 ml 5 mol/l HCL, $H_2O$, gesättigter $NaHCO_3$-Lösung und $H_2O$ gewaschen. Die organische Phase wird dann über $Na_2SO_4$ getrocknet und mit Aktivkohle unter Rückfluß zum Sieden erhitzt. Nach dem Abfiltrieren der Aktivkohle wird das Filtrat im Vakuum bis zur Trockne eingeengt. Das Produkt kann ohne weitere Reinigung für die nächste Stufe eingesetzt werden.

| | |
|---|---|
| Ausbeute: | 140 g (97 % d. Th.) |
| DC: | Kieselgel 60 $F_{254}$ (Merck) |
| | Toluol/Aceton (7/4) |
| | rf = 0.42 |
| $^1$H-NMR (DMSO-$d_6$): | 1.8 - 2.2 (s, 48H $CH_3CO$) |
| | 3.8 - 5.5 (m, 35H, H-1 - H-6) |
| | 5.7 (s, 1H, OH) |

Literatur:

Helferich, B.; Portz, W.; Chem. Ber. 86, (1953), 604.

Folgende Maltooligosaccharide wurden analog dieser Vorschrift synthetisiert:

| Zucker | Ausbeute | rf (Kieselgel 60, Toluol : Aceton, 7 : 4) |
|---|---|---|
| Maltose | 96 % | 0.45 |
| Maltotriose | 96 % | 0.40 |

**Beispiel 3**

**Peracetyl-maltopentaosyl-α-D-trichloracetimidat**

75 g (0.05 mol) Hydroxy-α-D-paracetyl-maltopentaosid und 25 ml (0.25 ml) Trichloracetonitril werden in 150 ml abs. $CH_2Cl_2$ gelöst. Die Lösung wird auf 0°C abgekühlt, unter Rühren portionsweise 1.5 g (0.055 mol) Natriumhydrid zugegeben und anschließend 2 h bei RT unter Feuchtigkeitsausschluß gerührt. Überschüssiges Natriumhydrid wird über einer Glasfritte abgetrennt, das Filtrat über einer Kieselgelsäule (60 Merck, ca. 200 ml, Ø 6 cm) filtriert und mit 2 l Essigester nachgewaschen. Das Filtrat wird mit Aktivkohle unter Rückfluß zum Sieden erhitzt, von der Aktivkohle abfiltriert und das Filtrat im Vakuum zur Trockne eingeengt.

| | |
|---|---|
| Ausbeute: | 72 g (87.5 % d. Th.) farbloses, schaumiges Produkt |
| DC: | Keiselgel 60 $F_{254}$ (Merck) |
| | Toluol/Aceton (7/4) |
| | rf = 0.58 |
| $^1$H-NMR (DMSO-$d_6$): | 1.8 - 2.2 (s, 48H, $CH_3CO$) |

3.8 - 5.5 (m, 35H, H-1 - H-6);
6.3 (s, 1H, NH)

Literatur:

Schmidt, R., Stumpp, M., Liebigs. Ann. Chem. 1983, 1249 - 1256

Analog dieser Vorschrift wurde noch Peracetyl-maltotriosetrichloracetimidat hergestellt.

| | |
|---|---|
| Ausbeute: | 93 % |
| rf: | 0.45 |

**Beispiel 4**

**Resorufinyl-β-D-maltopentaosid**

72 g (O,044 mol) Peracetylmaltopentaosyl-α-D-trichloracetimidat und 4.4 g (O.O2 mol) Resorufin werden in 1 l abs. DMF suspendiert. Unter $BF_3$.OEt-Katalyse wird unter Feuchtigkeitsausschluß 8 h bei 6O °C gerührt. Nach Abkühlen auf Raumtemperatur (RT) wird über $Al_2O_3$ (ca. 5OO ml) filtriert und mit 5 l Essigester nachgewaschen. Das Filtrat wird im Vakuum bis zur Trockne eingeengt, der Rückstand in 1OO ml abs. MeOH gelöst und mit 2 g $NaOCH_3$ 3 h bei RT unter Feuchtigkeitsausschluß deacetyliert. Die Suspension wird im Vakuum bis zur Trockne eingeengt, der Rückstand in 1OO ml $H_2O$ aufgenommen, mit 2 mol/l CHI auf pH = 6 gestellt und auf eine Diaion-Säule (ca. 5OO ml/Ø 8 cm) aufgezogen. Es wird zuerst mit 4 l $H_2O$, dann mit 3 l 2O %iger Isopropanol-Lösung eluiert. Das Eluat wird auf ca. 4O ml eingeengt und über eine RP-18-Flash-Säule (Ø = 4 cm, h = 39 cm) mit 15 % Isopropanol als Elutionsmittel in 5O ml-Portionen chromatographiert. Die Fraktion, die das Resorufinyl-maltopentaosid enthalten, werden vereinigt, auf ca. 2O ml in Vakuum eingeengt und über einer RP-18-MPLC-Säule in 1O ml-Portionen mit 15 % Isopropanol als Elutionsmittel chromatographiert. Die Fraktionen, die das Produkt enthalten, werden vereinigt, im Vakuum eingeengt und anschließend lyophilisiert.

| | |
|---|---|
| Ausbeute: | 2,8 g (14 % d. Th.) oranges Lyophilisat |
| HPLC: | RP-18-Säule, 1 ml/min, 17 % Isopropanol, rt = 2.95 min, Gehalt Resorufin-$G_5$: 97 % |
| $^1$H-NMR (DMSO-$d_6$): | 3.0 - 6.0 (m, 51H, OH, H-1 - H-6);<br>6.2 - 8.0 (m, 6H, ArH) |

Literatur:

Schmidt, R.; Grundler, G.; Synthesis 1981, 885 - 887

**Beispiel 5**

**Herstellung von Resorufinyl-β-D-maltooligosacchariden über die Königs-Knorr-Methode:**

1. Acetobrom-α-D-maltooligosaccharide:

25 mmol des peracetylierten Zuckers werden in 50 ml Eisessig gelöst. Dazu läßt man 50 ml HBr (30 %ig) in Eisessig hinzutropfen und rührt bei RT unter Feuchtigkeitsausschluß. Anschließend werden 300 ml $CH_2Cl_2$ hinzugefügt und auf 1 l Eiswasser gegossen. Die organsiche Phase wird abgetrennt und nacheinander mit $H_2O$, gesättigter $NaHCO_3$-Lösung und $H_2O$ ausgeschüttelt. Die organsiche Phase wird über $Na_2SO_4$ getrocknet und dann im Vakuum zur Trockne eingeengt. Das Produkt kann ohne weitere Aufreinigung in die Folgereaktion eingesetzt werden.

| Zucker | Ausbeute | rf (Kieselgel 60/Toluol : Aceton = 7 : 4) |
|---|---|---|
| Maltose | 88 % | O,72 |
| Maltotriose | 88 % | O,65 |
| Maltotetraose | 93 % | O,61 |
| Maltopentaose | 91 % | O,57 |
| Maltohexaose | 93 % | O,53 |
| Maltoheptaose | 95 % | O,50 |

Literatur:

Brauns, J. Am. Chem. Soc. 51, (1929), 1830

2. Resorufinyl-$\beta$-D-maltooligosaccharide:

50 mmol Resorufin und 25 mmol $Ag_2O$ werden in 800 ml abs. $CH_3CN$ mit Molekularsieb (3 Å) suspendiert und 4 h unter Feuchtigkeitsausschluß am Rückfluß gekocht. Man gibt 62. 5 mmol des Acetobrom-$\alpha$-D-maltooligosaccharides in 200 ml abs. $CH_3CN$ hinzu und läßt 6 h am Rückfluß kochen. Man läßt weitere 18 h bei RT rühren, setzt 10 g Aktivkohle hinzu und kocht kurz am Rückfluß. Das Gemisch wird über 100 g $Al_2O_3$ (Aktivitätsstufe III/N) filtriert und mit 5 l Essigester nachgewaschen. Das Filtrat wird im Vakuum bis zur Trockne eingeengt, der Rückstand in 1 l abs. Ethanol gelöst und mit 5 g $NaOCH_3$ unter Feuchtigkeitsausschluß bei RT 18 h gerührt. Die Suspension wird zur Trockne im Vakuum eingeengt, der Rückstand in 200 ml $H_2O$ gelöst, auf pH 7 eingestellt und auf eine Säule, gefüllt mit 500 ml Diaion-HP-20, aufgetragen. Es wird mit 15 l $H_2O$ gewaschen und mit 10 l einer 15 %igen Isopropanol-Lösung eluiert. Das Eluat wird auf 100 ml im Vakuum eingeengt und entsprechend der Imidat-Methode auf einer MPLC-Säule (RP-18) mit Isopropanol-Lösung chromatographiert und lyophilisiert.

| Zucker | Ausbeute | HPLC rt/% Isopropanol | rf (Kieselgel; Butanol/Eisessig/$H_2O$ 50/15/25) |
|---|---|---|---|
| Maltotriose[a)] | 15 % | 3.52 min/17 % | 0.32 |
| Maltotetraose | 16 % | 3.27 min/17 % | 0,29 |
| Maltopentaose | 14 % | 2.95 min/17 % | - |
| Maltoheptaose | 10 % | 2.60 min/17 % | - |
| Maltose[b)] | 15 % | - | 0.39 |

a) Bei Resorufinyl-ß-D-maltotriose Elution von Diaion-Säule mit 25 %igen Isopropanol und Chromatographie auf einer RP-18-Säule mit 25 %igem Isopropanol.

b) Bei Resorufinyl-ß-D-maltosid Elution von Diaion-Säule mit 40 %igem Isopropanol. Das Produkt wird im Vakuum zur Trockne einge dampft, dann in DMF gelöst und mit $H_2O$ ausgefällt. Der Niederschlag wird abgesaugt und im Vakuum getrocknet.

| | |
|---|---|
| $^1$H-NMR (DMSO-$d_6$): | |
| Maltose: | 3.1 - 3.9 (m, 11H, OH, H-6); |
| | 4.4 - 5.7 (m, 10H, H-1 - H-5); |
| | 6.2 - 8.0 (m, 6H, ArH) |
| Maltotriose: | 3.0 - 5.9 (m, 32H, OH, H-1 - H-6); |
| | 6.2 - 8.0 (m, 6H, ArH) |

## Beispiel 6

### Synthese geschützter Resorufinyl-β-D- und p-Nitrophenyl-α-D-maltooligosaccharide

1 mmol Resorufinyl- bzw. p-Nitrophenyl-maltooligosaccharid und 4 mmol Orthoester bzw. Acetal werden in 10 ml abs. DMF mit Molekularsieb (3 Å, frisch, aktiviert) unter Feuchtigkeitsausschluß gelöst. Dazu gibt man unter Rühren 1 mmol p-Toluolsulfonsäure und läßt 4 h bei RT rühren. Anschließend setzt man 20 ml $H_2O$ zu, läßt 10 min rühren und filtriert über DEAE-Sephacel ($CO_3^{2-}$). Man wäscht mit 20 %igem Isopropanol nach, engt das Filtrat auf 20 ml im Vakuum ein und chromatographiert fraktioniert über eine RP-18-MPLC-Säule mit 18 - 60 %igem Isopropanol. Die Produktfraktionen werden vereinigt, im Vakuum eingeengt und lyophilisiert. Im Falle der geschützten Verbindungen von Resorufinyl-β-D-maltosid fällt man aus DMF/$H_2O$. Die jeweils eingesetzten Verbindungen und die physikalischen Daten der Produkte ergeben sich aus Tabelle I.

## Beispiel 7

### Synthese endgruppen-geschützter Maltooligosaccharide nach der Mitsunobu-Reaktion

1 mmol Resorufinyl-maltopentaosid werden in abs. DMF mit 2 mmol der jeweiligen Carbonsäure, Triphenylphosphin und Diethylazodicarboxylat versetzt und 16 h bei RT unter Feuchtigkeitsausschlußgerührt. Darauf wird 20 ml $H_2O$ zugegeben und der entstandene Niederschlag über einen Seitz-Filter abfiltriert. Das Filtrat wird im Vakuum auf ca. 8 ml eingeengt und über eine RP-18-MPLC-Säule mit 18 % Isopropanol chromatographiert. Die Produktfraktionen werden vereinigt, im Vakuum eingeengt und lyophilisiert. Die jeweiligen Produkte sind mit den über die Orthoester-Methode (Beispiel 6) hergestellten Produkten identisch und zeigen die gleichen physikalischen Daten (Tabelle I).

**Tabelle I**

| Produkt | rt (HPLC, RP-18, 1 ml/min), Ret.Zeit/% Isoprop. | rf (DC, Kieselgel/ Butanol/Eisessig/Wasser 50 : 15 : 25) | $^1$H-NMR ($DMSO-d_6$) | eingesetzter Orthoester bzw. Acetal (Schutzgruppe) | % Isopropanol bei Chromatographie | Ausbeute |
|---|---|---|---|---|---|---|
| Resorufinyl-ß-D-maltopentaosyl-formiat | 3.19 min/17 % | --- | 3.2-5.6 (m, 49H, $CH_2$, OH, H-1-H-6) 5.0 (s, 1H, HCOO) 7.0-8.0 (m, 6H, Ar-H) | Trimethyl-orthoformiat | 20 % | 38 % |
| Resorufinyl-ß-D-maltopentaosyl-(N,N-dimethyl-amino)formiat | 3.19 min/17 % | --- | 2.9 (s, 6H, $CH_3$); 3.2-5.6 (m, 49H, $CH_2$, OH, H-1-H-6) 6.5-7.4 (m, 6H, Ar-H) | Trimethyl-ortho-N,N-dimethylamino-formiat** | 20 % | 15 % |
| Resorufinyl-ß-D-maltotriosyl-acetat | 3.50 min/20 % | 0.4 | --- | Trimethyl-orthoacetat | 25 % | 20 % |
| Resorufinyl-ß-D-maltotetraosyl-acetat | 3.40 min/17 % | 0.36 | --- | Trimethyl-ortho acetat | 17 % | 35 % |

* hergestellt nach DP 1122936

**Tabelle I** (Fortsetzung)

| Produkt | rt (HPLC, RP-18, 1 ml/min), Ret.Zeit/% Isoprop. | rf (DC, Kieselgel/ Butanol/Eisessig/Wasser 50 : 15 : 25) | $^1$H-NMR ($DMSO-d_6$) | eingesetzter Orthoester bzw. Acetal (Schutzgruppe) | % Isopropanol bei Chromatographie | Ausbeute |
|---|---|---|---|---|---|---|
| Resorufinyl-ß-D-maltopentaosyl-acetat | 3.16 min/17 % | --- | --- | Trimethyl-ortho-acetat | 15 % | 40 % |
| Resorufinyl-ß-D-maltoheptaosyl-acetat | 3.04 min/17 % | --- | --- | Trimethyl-ortho-acetat | 17 % | 25 % |
| Resorufinyl-ß-D-maltopentaosyl-propionat | 3.85 min/17 % | --- | --- | Trimethyl-ortho-propionat | 20 % | 25 % |
| Resorufinyl-ß-D-maltopentaosyl-butyrat | 3.30 min/25 % | --- | --- | Trimethyl-ortho-butyrat | 20 % | 20 % |
| Resorufinyl-ß-D-maltotetraosyl-isobutyrat | 4.20 min/17 % | --- | --- | Trimethyl-ortho-isobutyrat* | 29 % | 30 % |

* wurde hergestellt nach Litaratur Houben-Weyl, Band VI/3, 300-305

**Tabelle I** (Fortsetzung)

| Produkt | rt (HPLC, RP-18, 1 ml/min), Ret.Zeit/% Isoprop. | rf (DC, Kieselgel/ Butanol/Eisessig/Wasser 50 : 15 : 25) | $^1$H-NMR ($DMSO-d_6$) | eingesetzter Orthoester bzw. Acetal (Schutzgruppe) | % Isopropanol bei Chromatographie | Ausbeute |
|---|---|---|---|---|---|---|
| Resorufinyl-ß-D-maltopentaosyl-isobutyrat | 3.40 min/25 %<br>4.73 min/17 % | --- | --- | Triethyl-ortho-isobutyrat* | 20 % | 24 % |
| Resorufinyl-ß-D-maltopentaosyl-cyclopropyl-carboxylat | 3.14 min/20 % | --- | 0.9-1.0 (m, 4H, $CH_2$);1.6 (d, 1H, CH); 3.2-5.6 (m, 49H, $CH_2$, OH, H-1-H-6); 6.5-7.4 (m, 6H, Ar-H) | Trimethyl-ortho-cyclopropyl-carboxylat* | 20 % | 15 % |
| Resorufinyl-ß-D-maltopentaosyl-cyclohexycarboxylat | 3.05 min/25 % | --- | 1.6 (s, 10H, $CH_2$); 2.0 (d, 1H, CH); 3.2-5.6 (m, 49H, $CH_2$, OH, H-1-H-6); 6.5-7.4 (m, 6H, Ar-H) | Trimethyl-ortho-cyclohexycarboxylat* | 30 % | 30 % |

* wurde hergestellt nach Literatur Houben-Weyl, Band VI/3, 300-305

**Tabelle I** (Fortsetzung)

| Produkt | rt (HPLC, RP-18, 1 ml/min), Ret.Zeit/% Isoprop. | rf (DC, Kieselgel/ Butanol/Eisessig/Wasser 50 : 15 : 25) | $^1$H-NMR ($DMSO-d_6$) | eingesetzter Orthoester bzw. Acetal (Schutzgruppe) | % Isopropanol bei Chromatographie | Ausbeute |
|---|---|---|---|---|---|---|
| Resorufinyl-ß-D-maltopentaosyl-valerianat | 3.62 min/40 % | --- | 0.7-1.0 (m, 3H, $CH_3$); 1.0-1.8 (m, 4H, $CH_2$); 2.1-2.4 (m, 2H, $CH_2CO$); 3.0-6.0 (m, 49H, OH, H-1-H-6); 6.2-8.0 (m, 6H, Ar-H) | Trimethyl-ortho-valerianat | 40 % | 15 % |
| Resorufinyl-ß-D-maltopentaosyl-pivalonat | --- | --- | 1.0-1.2 (m, 9H, $CH_3$); 3.0-6.0 (m, 49H, $CH_2$, OH, H-1-H-6); 6.2-8.0 (m, 6H, Ar-H) | Trimethyl-ortho-pivalonat* | 25 % | 10 % |
| Resorufinyl-ß-D-maltopentaosyl-benzoat | 2.70 min/40 % | --- | 2.8-5.4 (m, 49H, H-1-H-6, OH); 6.2-8.0 (m, 11H, Ar-H) | Trimethyl-ortho-benzoat* | 25 % | 10 % |

* wurde hergestellt nach Literatur Houben-Weyl, Band VI/3, 300-305

**Tabelle I** (Fortsetzung)

| Produkt | rt (HPLC, RP-18, 1 ml/min), Ret.Zeit/% Isoprop. | rf (DC, Kieselgel/ Butanol/Eisessig/Wasser 50 : 15 : 25) | $^1H$-NMR ($DMSO-d_6$) | eingesetzter Orthoester bzw. Acetal (Schutzgruppe) | % Isopropanol bei Chromatographie | Ausbeute |
|---|---|---|---|---|---|---|
| p-Nitrophenyl-α-D-maltoheptaosyl-acetat | 4.16 min/13 % | --- | --- | Trimethyl-ortho-acetat | 15 % | 20 % |
| p-Nitrophenyl-α-D-maltoheptaosyl-isobutyrat | 5.50 min/13 % | --- | --- | Trimethyl-ortho-isobutyrat* | 15 % | 15 % |
| 4,6-Ethyliden-Resorufinyl-β-D-maltosid | --- | 0.62 | --- | Acetaldehyd-di-methylacetal | wurde aus DMF mit $H_2O$ gef. | 32 % |
| 4,6-Ethyliden-Resorufinyl-β-D-maltopentaosid | 2.53 min/40 % | --- | --- | Acetaldehyd-di-methylacetal | 20 % | 30 % |

* wurde hergestellt nach Literatur Houben-Weyl, Band VI/3, 300-305

**Tabelle I** (Fortsetzung)

| Produkt | rt (HPLC, RP-18, 1 ml/min), Ret.Zeit/% Isoprop. | rf (DC, Kieselgel/ Butanol/Eisessig/Wasser 50 : 15 : 25) | $^1$H-NMR (DMSO-$d_6$) | eingesetzter Orthoester bzw. Acetal (Schutzgruppe) | % Isopropanol bei Chromatographie | Ausbeute |
|---|---|---|---|---|---|---|
| Resorufinyl-ß-D-maltopentaosyl-thiophenyl-2-carboxylat | 5.08 min/17 % | --- | 3.2-5.6 (m, 49H, $CH_2$, OH, H-1-H-6) 7.0-8.0 (m, 9H, Ar-H) | Trimethyl-ortho-thiophenyl-2-carboxylat* | 20 % | 24 % |
| Resorufinyl-ß-D-maltopentaosyl-2-methoxypropionat | 6.73 min/17 % | --- | 0.9-1.0 (d, 3H, $CH_3$) 3.2-5.6 (m, 54H, $CH_2$, OH, CH, H-1-H-6, $CH_3O$) 7.0-8.0 (m, 6H, Ar-H) | Trimethyl-ortho-2-methoxy-propionat* | 20 % | 15 % |

* wurde hergestellt nach Literatur Houben-Weyl, Band VI/3, 300-305

## Beispiel 8

**α-Amylasebestimmung**

Reagenz (Endkonzentrationen im Test):
1 mmol/l Substrat (vgl. Tabelle II)
100 mmol/l HEPES-Puffer pH 7,1
50 mmol/l NaCl
10 mmol/l $MgCl_2$
30 U/l α-Glucosidase
1 U/l β-Glucosidase
1 ml Reagenz und 0,25 ml Probe pathologisches Humanserum werden zusammengegeben und das Gemisch auf 25 °C temperiert. Nach einer Vorinkubationszeit von 4 min (Lag-phase) wurde die Extinktionszunahme bei 578 nm an einem Photometer registriert und die Extinktionsänderung pro Minute (ΔE/min) bestimmt. Die Ergebnisse für die erfindungsgemäßen Substrate (1), (2), (4) und (6) sowie für die Vergleichsvderbindungen (3) und (5) sind aus Tabelle II zu ersehen:

Tabelle II

| Substrat | Leerwert ΔE/min | Probe ΔE/min |
|---|---|---|
| 1) Resorufinyl-β-D-$G_5$-acetat | 0,0140 | 0,535 |
| 2) Resorufinyl-β-D-$G_5$-isobutyrat | 0,0091 | 0,590 |
| 3) Resorufinyl-β-D-$G_5$ | 0,0182 | 0,430 |
| 4) Resorufinyl-β-D-$G_5$-propionat | 0,0111 | 0,408 |
| 5) 4,6-Ethyliden-resorufinyl-β-D-$G_5$ | 0,0018 | 0,0156 |
| 6) Resorufinyl-β-D-$G_5$-butyrat | 0,0038 | 0,191 |
| $G_5$: Maltopentaose | | |

**Patentansprüche**
**Patentansprüche für folgende Vertragsstaaten : AT, BE, GB, FR, DE, NL, IT, LU, SE, CH, LI**

1. Verbindungen der allgemeinen Formel I

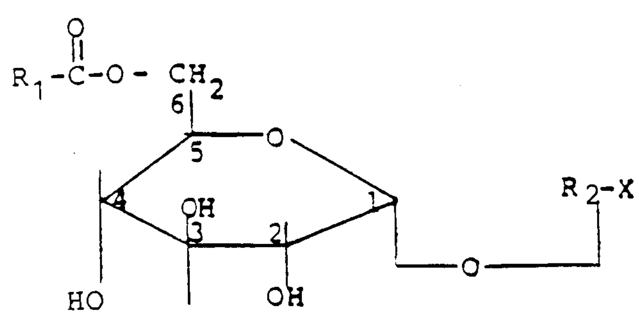

(I)

in der $R_1$ ein Wasserstoffatom, eine geradkettige oder verzweigte Alkylgruppe mit 1 bis 4 Kohlenstoffatomen, eine 1-Alkoxyalkyl- oder eine ggf. hydrophil substituierte Cycloalkylgruppe mit 3 bis 6 Kohlenstoffatomen, eine Phenyl-, Tetrahdropyranyl-, Piperidinyl-, ggf. N-Methyl- oder Ethyl-substituiert, Pyridinyl-, Thiophenyl, 1.1.-Dioxo-tetrahydrothiopyranyl-Gruppe oder eine Aminogruppe, die ggf. gleich oder verschieden substituiert ist mit einer Methyl-, Ethyl-, Propyl- oder Isopropylgruppe, $R_2$ einen Oligoglucosidrest mit 2, 3 oder 4 Glucoseeinheiten und X ein Wasserstoffatom oder einen optisch bestimmbaren Rest darstellen.

2. Verbindungen nach Anspruch 1, dadurch gekennzeichnet, daß $R_1$ eine Methyl-, Isopropyl- oder eine ggf. hydrophil substituierte Cyclopropylgruppe bedeutet.

3. Verbindungen nach Anspruch 1 und 2, dadurch gekennzeichnet, daß $R_2$ ein Oligoglucosidrest mit 4 Glucoseeinheiten ist.

4. Verbindungen nach Anspruch 1, 2 und 3, dadurch gekennzeichnet, daß X eine ggf. substituierte Nitrophenyl- oder Resorufingruppe darstellt.

5. Verfahren zur Bestimmung von α-Amylase durch Reaktion der Probe mit einer Verbindung der allgemeinen Formel I und α-Glucosidase und/oder β-Glucosidase und Bestimmung der Spaltprodukte.

6. Verfahren zur Bestimmung von α-Amylase nach Anspruch 5, dadurch gekennzeichnet, daß Glucoamylase zugesetzt wird.

7. Reagenz zur Bestimmung der α-Amylase, bestehend aus:
0,5 - 2 mmol/l Verbindung der allgemeinen Formel I
30 - 100 mmol/l NaCl
20 - 30 U/l α-Glucosidase und/oder
0,5 - 2 U/l β-Glucosidase
20 - 200 mmol/l Puffer pH 6 - 8

8. Reagenz nach Anspruch 7, dadurch gekennzeichnet, daß es 5 - 20 U/ml Glucoamylase enthält.

9. Reagenz nach Anspruch 7 oder 8, dadurch gekennzeichnet, daß es 5 - 20 mmol/l $MgCl_2$ enthält.

**Patentansprüche für folgende Vertragsstaaten : GR, ES**

1. Verfahren zur Herstellung von Verbindungen der allgemeinen Formel

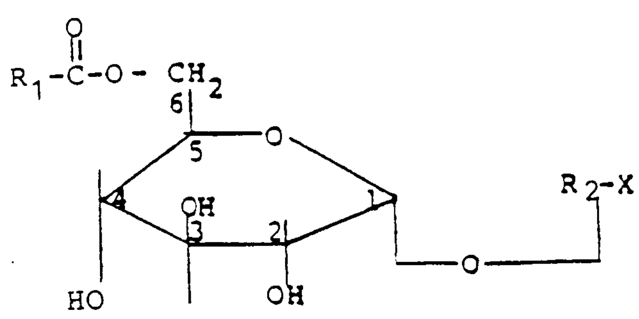

(I)

in der $R_1$ ein Wasserstoffatom, eine geradkettige oder verzweigte Alkylgruppe mit 1 bis 4 Kohlenstoffatomen, eine 1-Alkoxyalkyl- oder eine ggf. hydrophil substituierte Cycloalkylgruppe mit 3 bis 6 Kohlenstoffatomen, eine Phenyl-, Tetrahydropyranyl-, Piperidinyl-, ggf. N-Methyl- oder Ethyl-substituiert, Pyridinyl-, Thiophenyl- oder 1.1.-Dioxo-tetrahydrothiopyranyl-Gruppe, oder eine Aminogruppe, die ggf. gleich oder verschieden substituiert ist mit einer Methyl-, Ethyl-, Propyl- oder Isopropylgruppe, $R_2$ einen Oligoglucosidrest mit 2, 3 oder 4 Glucoseeinheiten und X ein Wasserstoffatom oder einen optisch bestimmbaren Rest darstellen, dadurch gekennzeichnet, daß man in an sich bekannter Weise Verbindungen der allgemeinen Formel II

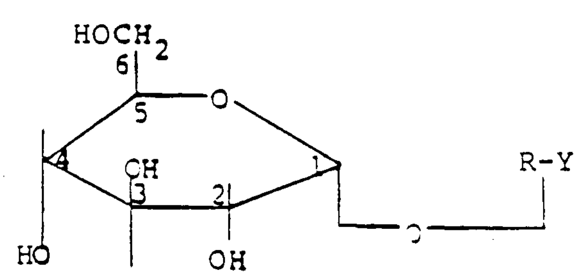

(II)

in der R ein Oligoglucosidrest mit 2, 3 oder 4 Glucoseeinheiten und Y ein Wasserstoffatom oder einen optisch bestimmbaren Rest darstellen, mit Carbonsäuren, deren Ester oder Orthoestern der allgemeinen Formel III

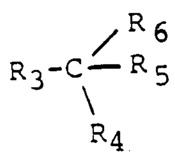

(III)

in der $R_3$ ein Wasserstoffatom, eine geradkettige oder verzweigte Alkylgruppe mit 1 bis 4 Kohlenstoffatomen, eine 1-Alkoxyalkyl- oder eine ggf. hydrophil substituierte Cycloalkygruppe mit 3 bis 6 Kohlenstoffatomen, eine Phenyl-, Tetrahydropyranyl-, Piperidinyl-, ggf. N-Methyl- oder Ethyl-substituiert, Pyridinyl-, Thiophenyl-, 1.1.-Dioxo-tetrahydrothiopyranyl-Gruppe oder eine Aminogruppe, die ggf. gleich oder verschieden substituiert ist mit einer Methyl-, Ethyl-, Propyl- oder Isopropylgruppe, $R_4$ eine Hydroxy-, Alkoxy-, eine Dimethyl- oder Diethyl-substituierte Aminogruppe, und $R_5$ und $R_6$, die gleich oder verschieden sein können, eine Alkoxygruppe mit 1 bis 4 Kohlenstoffatomen oder zusammen ein Sauerstoffatom darstellen, unter Feuchtigkeitsausschluß in wasserfreiem Lösungsmittel und Säurekatalyse oder in Gegenwart eines wasserentziehenden Mittels umsetzt.

**2.** Verfahren nach Anspruch 1, dadurch gekennzeichnet, daß man als Verbindung der allgemeinen Formel II Oligoglucoside mit einem ggf. substituierten Nitrophenyl- oder Resorufinrest und 5 Glucoseeinheiten verwendet.

**3.** Verfahren nach Anspruch 1 oder 2, dadurch gekennzeichnet, daß man als Orthoester Trimethyl- oder Triethylformiat, -acetat, -propionat, -butyrat, -isobutyrat, -cyclopropionat, -cyclohexylat, -valerianat, -pivalonat, -benzoat, -thiophenyl-2-carboxylat, -2-methoxypropionat oder -N,N-dimethylaminoformiat oder als Carbonsäure, Essigsäure, Propionsäure, Buttersäure, Valeriansäure, Pivalinsäure, ggf. hydrophil substituierte Cyclopropylcarbonsäure bzw. deren aktivierte Ester verwendet.

**4.** Verfahren nach Anspruch 1, 2 oder 3, dadurch gekennzeichnet, daß man als sauren Katalysator organsiche Säuren, Mineralsäuren und/oder Lewis-Säuren verwendet.

**5.** Verfahren nach Anspruch 1, 2 oder 3, dadurch gekennzeichnet, daß man als wasserentziehendes Mittel Triphenylphosphin und/oder Diethylazodicarboxylat verwendet.

**6.** Verfahren zur Bestimmung von $\alpha$-Amylase durch Reaktion der Probe mit einer Verbindung der allgemeinen Formel I und $\alpha$-Glucosidase und/oder $\beta$-Glucosidase und Bestimmung der Spaltprodukte.

**7.** Verfahren zur Bestimmung von $\alpha$-Amylase nach Anspruch 6, dadurch gekennzeichnet, daß Glucoamylase zugesetzt wird.

**8.** Verfahren zur Herstellung von Reagentien zur Bestimmung der $\alpha$-Amylase, dadurch gekennzeichnet, daß man 0,5 bis 2 mmol/l einer Verbindung der allgemeinen Formel I in 20 bis 200 mmol/l Puffer mit pH 6 bis 8 und 30 bis 100 mmol/l NaCl löst und 20 bis 50 U /l $\alpha$-Glucosidase und/oder 0,5 bis 2 U /l $\beta$-Glucosidase zusetzt.

**9.** Verfahren nach Anspruch 8, dadurch gekennzeichnet, daß man 5 bis 20 U/ml Glucoamylase zusetzt.

**10.** Verfahren nach Anspruch 8 oder 9, dadurch gekennzeichnet, daß man 5 bis 20 mmol/l $MgCl_2$ zusetzt.

**Claims**

**Claims for the following Contracting States : AT, BE, GB, FR, DE, NL, IT, LU, SE, CH, LI**

1. Compounds of the general formula I

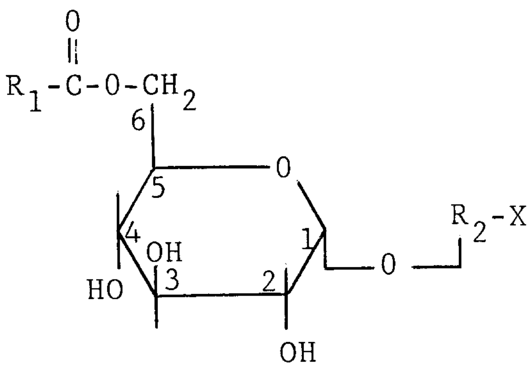

(I)

in which $R_1$ represents a hydrogen atom, a straight-chained or branched alkyl group with 1 to 4 carbon atoms, a 1-alkoxyalkyl or a possibly hydrophilically substituted cycloalkyl group with 3 to 6 carbon atoms, a phenyl, tetrahydropyranyl, piperidinyl, possibly N-methyl or ethyl-substituted, pyridinyl, thiophenyl, 1,1-dioxotetrahydrothiopyranyl group or an amino group which is possibly substituted the same or different with a methyl, ethyl, propyl or isopropyl group, $R_2$ an oligoglucoside residue with 2, 3 or 4 glucose units and X a hydrogen atom or an optically determinable residue.

2. Compounds according to claim 1, characterised in that $R_1$ signifies a methyl, isopropyl or a possibly hydrophilically substituted cyclopropyl radical.

3. Compounds according to claim 1 or 2, characterised in that $R_2$ is an oligoglucoside residue with 4 glucose units.

4. Compounds according to claim 1, 2 and 3, characterised in that X represents a possibly substituted nitrophenyl or resorufine radical.

5. Process for the determination of α-amylase by reaction of the sample with a compound of the general formula I and α-glucosidase and/or β-glucosidase and determination of the cleavage product.

6. Process for the determination of α-amylase according to claim 5, characterised in that glucoamylase is added thereto.

7. Reagent for the determination of α-amylase, consisting of
   0.5 - 2 mmol/l compound of the general formula I
   30 - 100 mmol/l NaCl
   20 - 50 U/l α-glucosidase and/or
   0.5 - 2 U/l β-glucosidase
   20 - 200 mmol/l buffer pH 6 - 8.

8. Reagent according to claim 7, characterised in that it contains 5 - 20 U/ml glucoamylase.

9. Reagent according to claim 7 or 8, characterised in that it contains 5 - 20 mmol/l $MgCl_2$.

**Claims for the following Contracting States : GR, ES**

**1.** Process for the preparation of compounds of the general formula

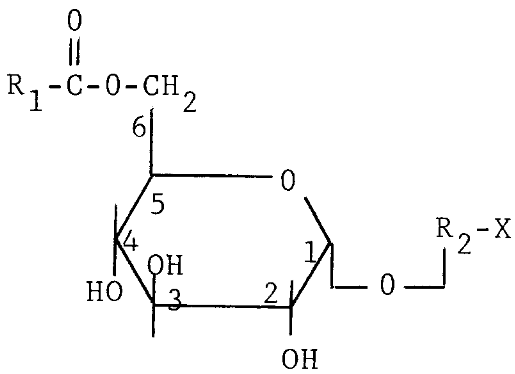

(I)

in which $R_1$ represents a hydrogen atom, a straight-chained or branched alkyl group with 1 to 4 carbon atoms, a 1-alkoxyalkyl or a possibly hydrophilically substituted cycloalkyl group with 3 to 6 carbon atoms, a phenyl, tetrahydropyranyl, piperidinyl, possibly N-methyl or ethyl substituted, pyridinyl, thiophenyl or 1,1-dioxotetrahydrothiopyranyl group or an amino group which is possibly substituted the same or different with a methyl, ethyl, propyl or isopropyl group, $R_2$ an oligoglucoside residue with 2, 3 or 4 glucose units and X a hydrogen atom or an optically determinable residue, characterised in that, in per se known way, one reacts compounds of the general formula II

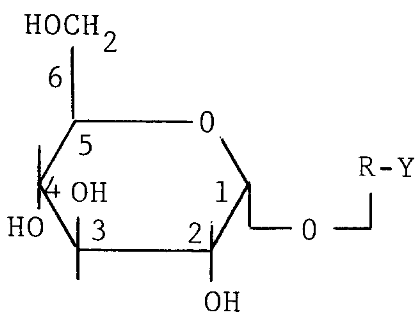

(II)

in which R represents an oligoglucoside residue with 2, 3 or 4 glucose units and Y a hydrogen atom or an optically determinable residue, with carboxylic acids, their esters or orthoesters of the general formula III

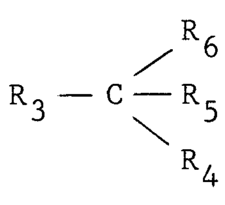

(III)

in which $R_3$ represents a hydrogen atom, a straight-chained or branched alkyl group with 1 to 4 carbon atoms, a 1-alkoxyalkyl or a possibly hydrophilically substituted cycloalkyl group with 3 to 6 carbon atoms, a phenyl, tetrahydropyranyl, piperidinyl, possibly N-methyl or ethyl substituted, pyridinyl, thiophenyl, 1,1-dioxotetrahydrothiopyranyl group or an amino group, which is possibly substituted the same or different with a methyl, ethyl, propyl or isopropyl group, $R_4$ a hydroxyl, alkoxy, a dimethyl- or diethyl-substituted amino group and $R_5$ and $R_6$, which can be the same or different, represent an alkoxy group with 1 to 4 carbon atoms or together an oxygen atom, with exclusion of moisture in anhydrous solvents and acid catalysis or in the presence of a water-removing agent.

2. Process according to claim 1, characterised in that, as compound of the general formula II, one uses oligoglucosides with a possibly substituted nitrophenyl or resorufin radical and 5 glucose units.

3. Process according to claim 1 or 2, characterised in that, as orthoester, one uses trimethyl- or triethyl-formate, -acetate, -propionate, -butyrate, -isobutyrate, -cyclopropionate, - cyclohexylate, -valerate, -pivalate, -benzoate, -thiophenyl-2-carboxylate, -2-methoxy-propionate or N,N-dimethylaminoformate or, as carboxylic acid, acetic acid, propionic acid, butyric acid, valeric acid, pivalic acid, possibly hydrophilically-substituted cyclopropylcarboxylic acid and their activated esters.

4. Process according to claim 1, 2 or 3, characterised in that, as acid catalysts, one uses organic acids, mineral acids and/or Lewis acids.

5. Process according to claim 1, 2 or 3, characterised in that, as water-removing agent, one uses triphenylphosphine and/or diethylazodicarboxylate.

6. Process for the determination of α-amylase by reaction of the sample with a compound of the general formula I and α-glucosidase and/or β-glucosidase and determination of the cleavage products.

7. Process for the determination of α-amylase according to claim 6, characterised in that glucoamylase is added.

8. Process for the preparation of reagents for the determination of α-amylase, characterised in that one dissolves 0.5 to 2 mmol/l of a compound of the general formula I in 20 to 200 mmol/l of buffer with pH 6 to 8 and 30 to 100 mmol/l NaCl and adds thereto 20 to 50 U/l of α-glucosidase and/or 0.5 to 2 U/l of β-glucosidase.

9. Process according to claim 8, characterised in that one adds thereto 5 to 20 U/ml glucoamylase.

10. Process according to claim 8 or 9, characterised in that one adds thereto 5 to 20 mmol/l of $MgCl_2$.

**Revendications**
**Revendications pour les Etats contractants suivants : AT, BE, GB, FR, DE, NL, IT, LU, SE, CH, LI**

1. Composés de formule générale (I)

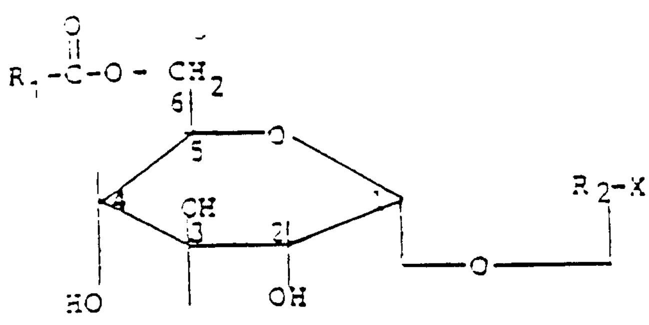

(I)

dans laquelle $R_1$ représente un atome d'hydrogène, un groupe alkyle à chaîne droite ou ramifiée comportant 1 à 4 atomes de carbone, un groupe 1 -alcoxyalkyle ou un groupe cycloalkyle, éventuellement à substitution hydrophile, comportant 3 à 6 atomes de carbone, un groupe phényle, tétrahydropyranyle, pipéridinyle, éventuellement substitué sur l'azote par un groupe méthyle ou éthyle, un groupe pyridinyle, thiophényle ou 1.1.-dioxo-tétrahydrothiopyranyle, ou un groupe amino qui est éventuellement substitué, par des substituants identiques ou différents, qui peuvent être un groupe méthyle, éthyle, propyle ou isopropyle, $R_2$ représente un résidu oligoglucosidique comportant 2, 3 ou 4 unités glucoside, et X représente un atome d'hydrogène ou un résidu optiquement détectable.

2. Composé selon la revendication 1, caractérisé en ce que $R_1$ représente un groupe méthyle, isopropyle ou un groupe cyclopropyle éventuellement à substitution hydrophile.

3. Composé selon la revendication 1 ou 2, caractérisé en ce que $R_2$ représente un résidu oligoglucosidique comportant 4 unités glucose.

4. Composé selon la revendication 1, 2 ou 3, caractérisé en ce que X représente un groupe nitrophényle ou résorufine éventuellement substitué.

5. Procédé pour la détermination d'α-amylase, par réaction de l'échantillon avec un composé de formule générale I et l'α-glucosidase et/ou la β-glucosidase, et détermination du produit de coupure.

6. Procédé pour la détermination d'α-amylase selon la revendication 5, caractérisé en ce que l'on ajoute de la glucoamylase.

7. Réactif pour la détermination d'α-amylase, composé de :
0,5 - 2 mmoles/l de composé de formule générale I,
30 - 100 mmoles/l de NaCl,
20 - 50 U/l d'α-glucosidase, et/ou
0,5 - 2 U/l de β-glucosidase,
20 - 200 mmoles/l de tampon, pH 6-8.

8. Réactif selon la revendication 7, caractérisé en ce qu'il contient 5 - 20 U/ml de glucoamylase.

9. Réactif selon la revendication 7 ou 8, caractérisé en ce qu'il contient 5 - 20 mmoles/l de $MgCl_2$.

**Revendications pour les Etats contractants suivants : GR, ES**

1. Procédé de préparation de composés de formule générale

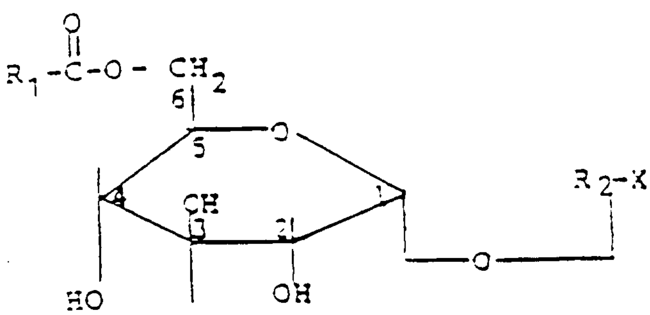

(I)

dans laquelle $R_1$ représente un atome d'hydrogène, un groupe alkyle à chaîne droite ou ramifiée comportant 1 à 4 atomes de carbone, un groupe 1-alcoxyalkyle ou un groupe cycloalkyle, éventuellement à substitution hydrophile, comportant 3 à 6 atomes de carbone, un groupe phényle, tétrahydropyranyle, pipéridinyle, éventuellement substitué sur l'azote par un groupe méthyle ou éthyle, un groupe pyridinyle, thiophényle ou 1.1.-dioxo-tétrahydrothiopyranyle, ou un groupe amino qui est éventuellement substitué, par des substituants identiques ou différents, qui peuvent être un groupe méthyle, éthyle, propyle ou isopropyle, $R_2$ représente un résidu oligoglucosidique comportant 2, 3 ou 4 unités glucoside, et X représente un atome d'hydrogène ou un résidu optiquement détectable,
caractérisé en ce que, de manière connue en soi, on fait réagir des composés de formule générale II

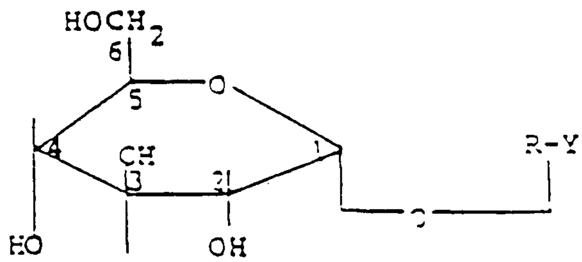

(II)

dans laquelle R représente un résidu oligoglucosidique comportant 2, 3 ou 4 unités glucose et Y représente un atome d'hydrogène ou un résidu optiquement détectable,
avec des acides carboxyliques, leurs esters ou orthoesters, de formule générale III

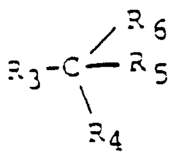

(III)

dans laquelle $R_3$ représente un atome d'hydrogène, un groupe alkyle à chaîne droite ou ramifiée comportant 1 à 4 atomes de carbone, un groupe 1-alcoxyalkyle ou un groupe cycloalkyle, éventuellement à substitution hydrophile, comportant 3 à 6 atomes de carbone, un groupe phényle, tétrahydropyranyle, pipéridinyle, éventuellement substitué sur l'azote par un groupe méthyle ou éthyle, un groupe pyridinyle, thiophényle ou 1.1.-dioxo-tétrahydrothiopyranyle, ou un groupe amino qui est éventuellement substitué, par des substituants identiques ou différents, qui peuvent être un groupe méthyle, éthyle, propyle ou isopropyle, $R_4$ représente un groupe hydroxyle, alcoxy, un groupe amino substitué par un groupe diméthyle ou diéthyle, et $R_5$ et $R_6$, qui peuvent être identiques ou différents, représentent un groupe alcoxy comportant 1 à 4 atomes de carbone, ou conjointement, représentent un atome d'oxygène,
à l'abri de l'humidité, dans un solvant anhydre et à l'aide d'un catalyseur acide, ou en présence d'un agent déshydratant.

2. Procédé selon la revendication 1, caractérisé en ce que, comme composé de formule générale II, on utilise des oligoglucosides comportant un résidu nitrophényle ou résorufine éventuellement substitué et 5 unités glucose.

3. Procédé selon la revendication 1 ou 2, caractérisé en ce que, comme orthoester, on utilise le formiate, l'acétate, le propionate, le butyrate, l'isobutyrate, le cyclopropionate, le cyclohexylate, le valérianate, le pivalonate, le benzoate, le thiophényl-2-carboxylate, le 2-méthoxypropionate ou le N,N-diméthylaminoformiate de triméthyle ou de triéthyle, ou comme acide carboxylique, on utilise l'acide acétique, l'acide propionique, l'acide butyrique, l'acide valérique, l'acide pivalique, l'acide cyclopropylcarboxylique éventuellement substitué par un groupe hydrophile, ou leurs esters activés.

4. Procédé selon la revendication 1, 2 ou 3, caractérisé en ce que, comme catalyseur acide, on utilise des acides organiques, des acides minéraux et/ou des acides de Lewis.

5. Procédé selon la revendication 1, 2 ou 3, caractérisé en ce que, comme agent déshydratant, on utilise la triphénylphosphine et/ou l'azodicarboxylate de diéthyle.

6. Procédé pour la détermination d'α-amylase$_7$ par réaction de l'échantillon avec un composé de formule générale I et l'α-glucosidase et/ou la β-glucosidase, et détermination du produit de coupure.

7. Procédé pour la détermination d'α-amylase selon la revendication 6, caractérisé en ce que l'on ajoute de la glucoamylase.

8. Procédé de préparation de réactifs pour la détermination d'α-amylase, caractérisé en ce que l'on dissout 0,5 à 2 mmoles/l d'un composé de formule générale I dans 20 à 200 mmoles/l de tampon, pH 6 à 8, et 30 à 100 mmoles/l de NaCl, et l'on ajoute 20 à 50 U/l d'α-glucosidase et/ou 0,5 à 2 U/l de β-glucosidase.

9. Procédé selon la revendication 8, caractérisé en ce que l'on ajoute 5 à 20 U/ml de glucoamylase.

10. Procédé selon la revendication 8 ou 9, caractérisé en ce que l'on ajoute 5 à 20 mmoles/l de $MgCl_2$